Europäisches Patentamt

European Patent Office

Office européen des brevets

(11) Veröffentlichungsnummer: **0 356 518**
**A1**

## EUROPÄISCHE PATENTANMELDUNG
### veröffentlicht nach Art. 158 Abs. 3 EPÜ

(21) Anmeldenummer: 88905036.5

(22) Anmeldetag: 23.02.88

(86) Internationale Anmeldenummer:
PCT/SU88/00042

(97) Internationale Veröffentlichungsnummer:
WO 89/08091 (08.09.89 89/21)

(51) Int. Cl.5: **C07C 11/08 , C07C 11/107 , C07C 2/30**

(43) Veröffentlichungstag der Anmeldung:
07.03.90 Patentblatt 90/10

(84) Benannte Vertragsstaaten:
**AT BE CH DE FR GB IT LI LU NL SE**

(71) Anmelder: **INSTITUT KHIMICHESKOI FIZIKI AKADEMII NAUK SSSR**
ul. Kosygina, 4
Moscow, 117977(SU)

Anmelder: **GROZNENSKY FILIAL OKHTINSKOGO NAUCHNO-PORIZVODSTVENNOGO OBIEDINENIA "PLASTPOLIMER"**

Grozny, 364048(SU)

Anmelder: **PROIZVODSTVENNOE OBIEDINENIE STAVROPOLPOLIMER**
Stavropolsky Krai
Budennovsk, 357920(SU)

(72) Erfinder: **ZHUKOV, Viktor Ivanovich**
ul. Neftezavodskaya, 4-1
Grozny, 364007(SU)
Erfinder: **BELOV, Gennady Petrovich** Shkolny bulvar, 5-53
Moskovskaya obl.
Chernogolovka, 142432(SU)
Erfinder: **SERGIENKO, Galina Stepanovna**
ul. Rabochaya, 64-43

Grozny, 364051(SU)
Erfinder: **DZHABIEVA, Zinaida Mikhailovna** proezd Stroitelei, 6-116 Moskovskaya obl.
Chernogolovka, 142432(SU)
Erfinder: **IVOLGINA, Saida Rakhimovna**
ul. B.Khmelnitskogo, 23-1
Grozny, 364013(SU)
Erfinder: **KARTASHEVA, Natalya Vasilievna**
mikroraion 7, 25 Stavropolsky krai
Budennovsk, 357920(SU)
Erfinder: **DYACHKOVSKY, Fridrikh Stepanovich**
ul. Kosygina, 6-9
Moscow, 117334(SU)
Erfinder: **IVANCHEV, Sergei Stepanovich**
ul. Nalichnaya, 36-37
Leningrad, 199226(SU)
Erfinder: **PROSKURNIN, Vladimir Leonidovich**
ul. Gudermesskaya, 96a-30
Grozny, 364015(SU)
Erfinder: **PETROV, Jury Maximovich**
mikroraion 7, 17-37 Stavropolsky krai
Budennovsk, 357920(SU)
Erfinder: **REZNIKOVA, Olga Nikolaevna**
mikroraion 7, 11-56 Stavropolsky krai
Budennovsk, 357920(SU)

(74) Vertreter: **von Füner, Alexander, Dr. et al**
Patentanwälte v. Füner, Ebbinghaus, Finck
Mariahilfplatz 2 & 3
D-8000 München 90(DE)

(54) **VERFAHREN ZUR HERSTELLUNG VON 1-BUTAN UND/ODER HEXENEN.**

(57) A method of obtaining l-butene and/or hexenes consists in dimerization of ethylene and/or codimerization of ethylene and propylene in an organic solvent at a high pressure and at a temperature of 0-100°C in the presence of a complex metal-organic catalyst containing titanium alcoholate of formula $Ti(OR)_4$, where R is an alkyl radical having 1 to 8 carbon atoms or aryl, trialkylaluminium of formula $AlR_3$, where R is an alkyl radical having 1 to 6 carbon atoms, a magnesium-organic compound of formula $MgR_2$ and/or MgRX, where R is an alkyl radical having 1 to 4 carbon atoms and X is Br, Cl, I.

# VERFAHREN ZUR HERSTELLUNG VON BUTEN-(1) UND/ODER HEXENEN

## Gebiet der Technik

Die vorliegende Erfindung bezieht sich auf die Petrolchemie und betrifft insbesondere ein Verfahren zur Herstellung von Buten-(1) und/oder Hexenen.

## Zugrundeliegender Stand der Technik

Bekannt ist ein Verfahren zur Herstellung von Buten-(1) oder Hexenen (SU, A, 653119) durch Dimerisation von Äthylen beziehungsweise Kodimerisation von Äthylen und Propylen in Gegenwart eines Katalysators, der sich aus Titanalkoholat der Formel $Ti(OR)_4$ und einer komplexen aluminiumorganischen Verbindung der Formel $Al\ R_3L$ zusammensetzt, worin R - Alkylrest ist, der von 1 bis 4 Kohlenstoffatomen hat, L - eine Verbindung ist, gewählt aus der Gruppe: Dialkylester, Dialkylamin, aliphatisches Diamin, Di(diphenylphosphino)äthylamin. Diäthylsilfid. Die Umsetzung der Monomere erfolgt in einem organischen Lösungsmittel bei einer Temperatur von 0 bis 80°C und einem Druck von 0,1 bis 20,0 MPa. Dieses Verfahren zeichnet sich durch eine niedrige Ausbeute an Endprodukt (bis 590 g/g $Ti(OR)_4$ und durch die Bildung eines festen Polymers (bis 0,1 Masse%) in der Reaktionszone aus. Bekannt ist ebenfalls ein Verfahren zur Herstellung von Buten-(1) (SU, A, 459451), das in der Dimerisation von Äthylen in Gegenwart eines komplexen metallorganischen Katalysators, der sich aus Tetraalkoxytitanat, Trialkylaluminium (Alk = $C_1$-$C_4$) und einem organischen Zusatzstoff: Isopropanol, Butanol oder Phenol zusammensetzt, in einem organischen Lösungsmittel bei einer Temperatur von 70 bis 80°C und einem Druck von 0,25 bis 1,4 MPa besteht.

Bei einer hohen Selektivität der Prozeßführung (bis 99,75 Vol.% an Buten-(1)) ist die Ausbeute an Buten-(1) nicht hoch und beträgt von 101 bis 214 g/g $Ti(OR)_4$ pro Stunde.

Bekannt ist auch ein Verfahren zur Herstellung von Buten-(1) und/oder Hexenen (US, A, 4, 101,600), das in der Dimerisation von Äthylen und/oder in der Kodimerisation von Äthylen und Propylen in Gegenwart eines komplexen metallorganischen Katalysators, der Titanalkoholat der Formel $Ti\ (OR)_4$,

- 2 -

worin R - Alkylrest, der von 1 bis 8 Kohlenstoffatomen hat, oder Aryl ist Trialkylaluminium der Formel Al R$_3$, worin R - Alkylrest ist, der von 1 bis 6 Kohlenstoffatomen hat, aufweist, bei einer Temperatur von 0 bis 100°C und einem erhöhten Druck im Medium eines organischen Lösungsmittel besteht. Der Katalysator in diesem Verfahren wird zur Erhöhung seiner Aktivität und Selektivität vorher mit einem Äthylen-Wasserstoff-Gemisch behandelt. Dieses Gemisch ist jedoch explosionsgefährlich, was die technologische Prozeßführung kompliziert und den Einsatz spezieller Ausrüstungen verlangt. Die Ausbeute an Buten-(1) ist nicht hoch und beträgt lediglich von 80 bis 347 g/g Ti (OR)$_4$ pro Stunde, obwohl der Gehalt an Hexenen im Endprodukt 25 Masse% beim Vorliegen eines festen Polymers in einer Menge bis 0,4 Masse% erreicht.

Offenbarung der Erfindung

Der Erfindung liegt die Aufgabe zugrunde, ein Verfahren zur Herstellung von Buten-(1) und/oder Hexenen mittels der qualitativen Veränderung des komplexen metallorganischen Katalysators zu entwickeln, welcher die Steigerung der Ausbeute an Endprodukt ohne Komplizierung der Technologie gewährleistet.

Die Aufgabe wird dadurch gelöst, daß ein Verfahren zur Herstellung von Buten-(1) und/oder Hexenen vorgeschlagen wird, das in der Dimerisation von Äthylen und/oder in der Kodimerisation von Äthylen und Propylen in Gegenwart eines komplexen metallorganischen Katalysators, der Titanalkoholat der Formel Ti(OR)$_4$, worin R - Alylrest, der von 1 bis 8 Kohlenstoffatomen hat oder Aryl ist, Trialkylaluminium der Formel Al R$_3$, worin R - Alkylrest ist, der von 1 bis 6 Kohlenstoffatomen enthält, aufweist, bei einer Temperatur von 0 bis 100°C und einem erhöhten Druck im Medium eines organischen Lösungsmittels besteht, in dem, erfindungsgemäß, man einen komplexen metallorganischen Katalysator verwendet, der zusätzlich eine magnesiumorganische Verbindung der Formel MgR$_2$ und/oder MgRX enthält, worin R- Alkylrest ist, der von 1 bis 4 Kohlenstoffatomen hat, X für Br, Cl, J steht. Die Verwendung eines solchen Katalysators führt zur Erhöhung der

- 3-

Ausbeute an Endprodukt bis 1300 g/g Ti(OR)$_4$ bei der Senkung der Ausbeute an Nebenprodukten (Okten und festem Polymer) auf das 2 bis 100fache.

Zweckmäßigerweise verwendet man für die Prozeßführung unter optimalen Betriebsbedingungen und für maximale Ausbeute an Endprodukt den genannte Katalysator, der Ti (OR)$_4$, Al R$_3$, Mg R$_2$ und/oder Mg RX bei einem Molverhältnis 1,0:1,0-100:0,2-5,0 enthält.

Für die Vereinfachung der technologischen Prozeßführung wird es empfohlen, als kohlenwasserstoffhaltiges organisches Lösungsmittel (Buten-(1) und/oder eine Hexenfraktion zu verwenden.

Beste Ausführungsvariante der Erfindung

In einen Reaktor führt man ein organisches Lösungsmittel ein, das Ti (OR)$_4$, Al R$_3$ und Mg R$_2$ und/oder Mg RX bei einem Molverhältnis 1,0:1,0-100:0,2-0,5 enthält, und man führt Äthylen und/oder ein Gemisch aus Äthylen und Propylen mit anschließender Durchführung der Dimerisation und/oder Kodimerisation bei einer Temperatur von 0 bis 100°C und einem Druck von 0,1 bis 4 MPa bis zum Anfallen von Buten-(1) und/oder einer Hexenfraktion zu, die (in Vol.%) enthält: 3-Methyl-1-penten; Hexen-(1); 2-Äthyl-1-Buten.

Als organisches Lösungsmittel kann man ein aliphatisches (Propan, Butan, Heptan, Hexan, Oktan), ein aromatisches (Benzol, Toluol), ein heteroatomhaltiges (Halogenalkyle, Äther und Ester) und ein kohlenwasserstoffhaltiges Lösungsmittel verwenden. Die größte Vereinfachung der technologischen Prozeßführung wird durch die Verwendung von Buten-(1) oder einer Hexenfraktion als organisches Lösungsmittel erreicht. Es wird vollständig die Stufe der Rückgewinnung von Lösungsmittel ausgeschlossen, dabei wird eine wesentliche Steigerung der Ausbeute an Endprodukt beobachtet.

In Gegenwart von Olefin bilden Ti (OR)$_4$, Al R$_3$, Mg R$_2$ und/oder Mg RX im organischen Lösungsmittel einen komplexen metallorganischen Katalysator. Die genannte magnesiumorganische Verbindung kann in die Reaktionszone gleichzeitig mit Ti (OR)$_4$ und Al R$_3$ oder nach der Zuführung von Olefin eingeführt werden.

Die Verwendung der magnesiumorganischen Verbindung als dritte Komponente des komplexen Katalysators, führt, erfindungsgemäß, zu einer wesentlichen Steigerung seiner Aktivität. Das bewirkt seinerseits eine wesentliche Erhöhung der Geschwindigkeit der Dimerisation und Kodimerisation bei der gleichzeitigen Vergrößerung ihrer Selektivität.

Neben einer wesentlichen Intensivierung der Prozeßführung erreicht die Ausbeute an Endprodukt 1300 g/g Ti $(OR)_4$ pro Stunde oder 9,2 kg/g Ti.

Nachstehend werden konkrete Beispiele für die Realisierung des vorgeschlagenen Verfahrens angeführt.

Beispiel 1

In einen Reaktor werden 150 ml Hektan, $0,256 \cdot 10^{-3}$ Mol Ti $(OC_4H_9)_4$, $0,96 \cdot 10^{-3}$ Mol Al $(C_2H_5)_3$ und $0,123 \cdot 10^{-3}$ Mol Mg $C_4H_9Cl$ bei ihrem Molverhältnis 1,0:4,0 bzw. 0,5 aufgegeben und man führt Äthylen zu. Die Dimerisation erfolgt bei einer Temperatur von 40°C und einem Äthylendruck von 0,7 MPa. Die Ausbeute am angefallenen Produkt beträgt 440 g/g Ti $(OC_4H_9)_4$ pro Stunde oder 3,1 kg/g Ti pro Stunde. Es hat folgende Zusammensetzung (in Vol.%): Buten-(1) - 96,4; Hexen-(1) - 0,1; 3-Methyl-1-penten - 1,4; 2-Äthyl-1-buten - 1,8; Polymer - Spuren.

Beispiel 2

Die Dimerisation von Äthylen erfolgt wie in Beispiel 1 bei einem Molverhältnis Ti $(OC_4H_9)_4$ :Al $(C_2H_5)_3$: Mg $C_4H_9Cl$ = 1,0:4,18:2,0. Die Ausbeute an Produkt beträgt 632 g/g Ti $(OC_4H_9)_4$ pro Stunde oder 4,8 kg/g Ti pro Stunde. Das angefallene Produkt hat folgende Zusammensetzung (in Vol. %): Buten-(1) - 96,9; Hexen-(1) - 0,2; 3-Methyl-1-penten - 1,1; 2-Äthylen-1-buten - 1,8; Polymer - Spuren.

Beispiel 3

Die Dimerisation von Äthylen erfolgt wie in Beispiel 2 bei einem Molverhältnis Ti $(OC_4H_9)_4$: Al $(C_2H_5)_3$ : Mg $C_4H_9Cl$ = 1,0:4,7:5,0.

Die Ausbeute am angefallenen Produkt beträgt 740 g/g Ti $(OC_4H_9)_4$ pro Stunde oder 5,2 kg/g Ti pro Stunde. Es hat folgende Zusammensetzung (in Voll.%): Buten-(1) - 97,6;

- 5 -

Hexen-(1) - 0,3; 3-Methyl-1-penten - 0,8; 2- Äthyl-1-buten - 1,3; Polymer - Spuren.

Beispiel 4

Die Dimerisation von Äthylen erfolgt bei einer Temperatur von 65°C wie in Beispiel 1. Als Mg RX verwendet man Mg $C_2H_5Br$. Das Molverhältnis

Ti $(OC_4H_9)$ : Al $(C_2H_5)_3$ : Mg $C_2H_5Br$ = 1,0:3,72:1,0.

Die Ausbeute am angefallenen Produkt beträgt 503 g/g Ti $(OC_4H_9)_4$ pro Stunde oder 3,6 kg/g Ti pro Stunde. Es hat folgende Zusammensetzung (in Vol.%): Buten-(1) - 96,9; Hexen-(1) - 0,4; 3-Methyl-1-penten - 1,1; 2- Äthyl-1-buten - 1,6; Polymer - Spuren.

Beispiel 5

Die Dimerisation von Äthylen erfolgt wie in Beispiel 1 mit Ausnahme dessen, daß man Mg $CH_3J$ verwendet und das Molverhältnis beträgt Ti $(OC_4H_9)_4$ : Al $(C_2H_5)_3$ : Mg $CH_3J$ = 1,0:4,0:0,5. Die Ausbeute am angefallenen Produkt beträgt 273 g/g Ti $(OC_4H_9)_4$ pro Stunde oder 1,9 kg/g Ti pro Stunde. Es hat folgende Zusammensetzung (in Vol.%): Buten-(1) - 97,8; Hexen-(1) - 0,2; 3-Methyl-1-penten - 0,5; 2-Äthyl-1-buten - 0,6; Polymer - Spuren.

Beispiel 6

In einen Reaktor bringt man 150 ml Hexandraktion, $0,256 \cdot 10^{-3}$ Mol Ti $(OC_4H_9)_4$, $0,752 \cdot 10^{-23}$ Mol Al $(C_2H_5)_3$ und $0,128 \cdot 10^{-23}$ Mol Mg $C_2H_5Br$ bei einem Molverhältnis 1,0:2,94:0,5 ein und man führt Äthylen zu. Die Dimerisation erfolgt bei einer Temperatur von 40°C und einem Druck von Äthylen von 0,7 MPa. Die Ausbeute von Buten-(1) beträgt 98 g/g Ti $(OC_4H_9)_4$ pro Stunde oder 0,695 kg/g Ti pro Stunde, die Selektivität der Prozeßführung beträgt nach Buten-(1) - 82,0 Vol.%

Beispiel 7

Die Dimerisation von Äthylen erfolgt wie in Beispiel 6 bei einem Molverhältnis Ti $(OC_4H_9)_4$ : Al $(iso-C_4H_9)_3$ : Mg $C_2H_4Br$ = 1,0:2,9:2,0. Die Ausbeute an Buten-(1) beträgt 95 g/g Ti $(OC_4H_9)_4$ pro Stunde oder 0,674 kg/g Ti pro Stunde bei einer Selektivität der Prozeßführung nach Buten-(1) gleich 87,4 Vol.%.

Beispiel 8

Die Dimerisation von Äthylen erfolgt wie in Beispiel 6 bei einem Molverhältnis

$Ti(OC_4H_9)_4 : Al(iso-C_4H_9)_3 : Mg C_2H_5Br = 1,0:4,0:4,0$.

Die Ausbeute an Buten-(1) beträgt 90 g/g $Ti(OC_4H_9)_4$ pro Stunde oder 0,639 kg/g Ti pro Stunde. Die Selektivität nach Buten-(1) beträgt 88,1 Vol.%.

Beispiel 9

Die Dimerisation von Äthylen erfolgt wie in Beispiel 1 mit Ausnahme dessen, daß man als $Mg R_2$ $Mg(C_2H_5)_2$ verwendet. Das Molverhältnis $Ti(OC_4H_9)_4 : Al(C_2H_5)_3 : Mg(C_2H_5)_2 = 1,0:3,72:1,0$. Die Ausbeute an Produkt beträgt 490g/g $Ti(OC_4H_9)_4$ pro Stunde oder 3,5 kg/g Ti pro Stunde. Das angefallene Produkt hat folgende Zusammensetzung (Vol.%): Buten-(1) - 96,1; Hexen-(1) - 0,4; 3-Methyl-1-penten - 1,6; 2-Äthyl-1-buten - 1,9; Polymer - Spuren.

Beispiel 10

Die Dimerisation von Äthylen erfolgt wie in Beispiel 1 mit Ausnahme dessen, daß man als $MgR_2$ $Mg(C_4H_9)_2$ verwendet. Das Molverhältnis $Ti(OC_4H_9)_4 : Al(C_2H_5)_3 : Mg(C_4H_9)_2 = 1,0:3,72:1,0$. Die Ausbeute an Produkt beträgt 445 g/g $Ti(OC_4H_9)_4$ pro Stunde oder 3,1 kg/g Ti pro Stunde. Das angefallene Produkt hat folgende Zusammensetzung (Vol.%): Buten-(1) - 96,0; Hexen-(1) - 0,1; 3-Methyl-1-penten - 1,6; 2-Äthyl-1-buten - 2,3; Polymer - Spuren.

Beispiel 11

In einen Reaktor bringt man 200 ml n-Heptan ein, das $1,45 \cdot 10^{-3}$ Mol $Ti(OC_6H_5)_4$; $2,2 \cdot 10^{-3}$ Mol $Al(C_2H_5)_3$ enthält, und man führt das Äthylen-Propylen-Gemisch bei ihrem Molverhältnis 1:1 zu. Dann führt man $2,9 \cdot 10^{-3}$ Mol Mg $C_2H_5Br$ ein. Das Molverhältnis $Ti(OC_6H_5)_4 : Al(C_2H_5)_3 : Mg C_2H_5Br = 1,0:3,4:2,0$. Die Kodimerisation erfolgt bei einer Temperatur von 30°C und einem Druck von 0,1 MPa innerhalb von 60 Minuten und sie wird durch Zusatz von Alkohol unterbrochen. Die Ausbeute an Produkt beträgt 2,3 g oder 191 g/g Ti pro Stunde. Das Produkt hat folgende Zusammensetzung (in Vol.%): Buten-(1) - 60,7; 3-Methyl-1-buten - 9,3; 2- Methyl-1-buten - 21,8; 3-Methyl-1-penten - 5,4; 2-Äthyl-1-buten - 2,8.

Beispiel 12

Die Kodimerisation von Äthylen und Propylen erfolgt wie in Beispiel 11 mit Ausnahme dessen, daß man als
Ti (OR)$_4$ Ti (OC$_4$H$_9$)$_4$. Das Molverhältnis
Ti (OC$_4$H$_9$)$_4$ : Al (C$_2$H$_5$)$_3$ : Mg (C$_2$H$_5$Br) - 1,0:3,4:2,0.
Die Ausbeute an Produkt beträgt 3,5 g oder 248 g/g Ti pro
Stunde. Das angefallene Produkt hat folgende Zusammensetzung (in Vol.%): Buten-(1) - 55; 3-Methyl-1-buten - 15;
2-Methyl-1-buten - 19; 3-Methyl-1-penten - 7,4;
2-Äthyl-1-buten - 3,6; Polymer - Spuren.

Beispiel 13

Die Dimerisation von Äthylen erfolgt bei einer Temperatur von 80°C, wie in Beispiel 1, bei einem Molverhältnis
Ti (OC$_4$H$_9$)$_4$ : Al (iso-C$_4$H$_9$)$_3$ : Mg C$_2$H$_5$Br = 1,0:2,0:0,3.
Die Ausbeute an Buten-1 beträgt 90 g/g Ti (OC$_4$H$_9$)$_4$ pro Stunde oder 639 g/g Ti pro Stunde. Die Selektivität der Prozeßführung nach Buten-(1) beträgt 69 Vol.%.

Beispiel 14

Die Dimerisation von Äthylen erfolgt im Buten-(1) bei
einer Temperatur von 65°C und einem Äthylendruck von 1,4 MPa.
Der Katalysator wird in Beispiel 1 bei einem Molverhältnis
Ti (OC$_4$H$_9$)$_4$ : Al (C$_2$H$_5$)$_3$ : Mg (C$_4$H$_9$Cl) = 1,0:4,7:5,0 verwendet. Die Ausbeute an Produkt beträgt 495 g/g Ti (OC$_4$H$_9$)$_4$ pro
Stunde oder 3,5 kg/g Ti pro Stunde. Das Produkt hat folgende
Zusammensetzung (in Vol.%): Buten-(1) -95,8; 3-Methyl-1-
penten - 1,8; Hexen-(1) - 0,2; 2-Äthyl-1-buten - 2,1; Olefinfraktion C$_8$-C$_{12}$ - 0,05; Polymer - 0,05.

Beispiel 15

Die Dimerisation von Äthylen erfolgt in einer Hexenfraktion, die (in Vol.%) enthält: 3-Methylen-1-penten - 33;
2-Äthyl-1-buten - 55; Hexen-(1) - 12; bei einer Temperatur
von 35°C und einem Druck von 1,4 MPa. Man verwendet den Katalysator wie in Beispielen 1 und 14.
Die Ausbeute an Produkt beträgt 460 g/g Ti (OC$_4$H$_9$)$_4$
pro Stunde oder 3,2 kg/g Ti pro Stunde. Es hat folgende Zusammensetzung in (Vol.%): Buten-(1) - 94,5; Hexene - 5,42;
Oktene und Polymer - 0,08.

Beispiel 16

In einen Reaktor werden 200 ml n-Heptan, das 1,6$\cdot$10$^{-3}$
Mol Ti (OC$_4$H$_9$)$_4$, 6,4$\cdot$10$^{-3}$ Mol Al (C$_2$H$_5$)$_3$, 1,6$\cdot$10$^{-3}$ Mol des

- 8 -

Gemisches von Mg $C_4H_9Cl$ und Mg $(C_4H_9)_2$ (3:1) aufgegeben und dann führt man Äthylen zu. Das Molverhältnis Ti $(OC_4H_9)_4$ : Al $(C_2H_5)_3$ : Gemisch von Mg $C_4H_9Cl$ und Mg $(C_4H_9)_2$ = 1,0: 3,84:1,0. Die Dimerisation erfolgt bei einer Temperatur von 55°C und einem Druck von 0,8 MPa. Die Ausbeute an Produkt beträgt 872 g/g Ti $(OC_4H_9)_4$ pro Stunde oder 6,2 kg /g Ti pro Stunde. Das Produkt hat folgende Zusammensetzung (in Vol.%): Buten-(1) - 99,4; 3-Methyl-1-penten - 0,2; Hexen-(1) - 0,1; 2-Äthyl-1-buten - 0,3.

Beispiel 17

Die Dimerisation von Äthylen erfolgt bei einer Temperatur von 30°C, wie in Beispiel 16, mit Ausnahme dessen, daß man als MgRX $MgCH_3J$ verwendet. Das Molverhältnis Ti $(OC_4H_9)_4$ : Al $(C_2H_5)_3$ : Mg $CH_3J$ = 1,0:4,0:0,2. Die Ausbeute an Produkt beträgt 469 g/g Ti $(OC_4H_9)_4$ pro Stunde oder 3,3 kg/g Ti pro Stunde.

Das Produkt hat folgende. Zusammensetzung (in Vol.%): Buten-(1) - 96,7; 3-Methyl-1-buten - 1,5; Hexen-(1) - 0,2; 2-Äthyl-1-buten - 1,6; Polymer - 0,01.

Beispiel 18

Die Dimerisation von Äthylen erfolgt bei einer Temperatur von 65°C, wie in Beispiel 16, mit Ausnahme dessen, daß man als $Mg_2$ Mg $(C_4H_9)_2$ verwendet. Das Molverhältnis Ti$(OC_4H_9)_4$ : Al $(C_2H_5)_3$ : Mg $(C_4H_9)_2$ = 1,0:92:0,50. Die Ausbeute an Produkt beträgt 767 g/g Ti $(OC_4H_9)_4$ pro Stunde oder 5,4 kg/g Ti pro Stunde. Das Produkt hat folgende Zusammensetzung (in Vol.%): Buten-(1) - 94,9; 3-Methyl-1-buten - 2,1; Hexen-(1) - 0,3; 2-Äthyl-1-buten - 2,7; Polymer - 0,2.

Beispiel 19

Die Dimerisation von Äthylen erfolgt bei einer Temperatur von : 80°C, wie in Beispiel 16, mit Ausnahme dessen, daß man als $MgR_2$ Mg $(C_4H_9)_2$ verwendet. Das Molverhältnis Ti $(OC_4H_9)_4$ : Al $(C_2H_5)_3$ : Mg $(C_4H_9)_2$ = 1,0:7,65:2,0. Die Ausbeute an Produkt beträgt 750 g/g Ti $(OC_4H_9)_4$ pro Stunde oder 5,3 kg/g Ti pro Stunde. Das Produkt hat folgende Zusammensetzung (in Vol.%): Buten-(1) - 94,2; 3-Methyl-1-buten - 2,2; Hexen-(1) - 0,5; 2-Äthylen-1-buten - 3,1.

Beispiel 20

Die Dimerisation von Äthylen erfolgt bei einer Temperatur von 25°C, wie in Beispiel 16, mit Ausnahme dessen, daß man als Mg RX ein Gemisch von Mg $C_2H_5Br$ und Mg $(C_2H_5)_2$ (4:1) verwendet. Das Molverhältnis Ti $(OC_4H_9)_4$ : Al $(C_2H_5)_3$ : Gemisch von Mg $C_2H_5Br$ und Mg $(C_2H_5)_2$ = 1,0:3,7:1,0. Die Ausbeute an Produkt beträgt 750 g/g Ti $(OC_4H_9)_4$ pro Stunde oder 5,3 kg/g Ti pro Stunde. Das Produkt hat folgende Zusammensetzung (in Vol.%): Buten-(1) - 96,9; 3-Methyl-1-buten - 1,1; Hexen-(1) - 0,4; 2-Äthyl-1-buten - 1,6.

Beispiel 21

Die Dimerisation von Äthylen erfolgt bei einer Temperatur von 65°C, wie in Beispiel 16, mit Ausnahme dessen, daß man als MgRX Mg $C_2H_5Br$ verwendet. Das Molverhältnis Ti $(OC_4H_9)_4$: Al $(C_2H_5)_3$ : Mg $C_2H_5Br$ = 1,0:3,7:1,0. Die Ausbeute an Produkt beträgt 872 g/g Ti $(OC_4H_9)_4$ pro Stunde oder 6,2 kg/g Ti pro Stunde. Das Produkt hat folgende Zusammensetzung (in Vol.%): Buten-(1) - 95,3; 3-Methyl-1-buten - 1,9; Hexen-(1) - 0,3; 2-Äthyl-1-buten - 2,5.

Beispiel 22

Die Dimerisation von Äthylen erfolgt bei einer Temperatur von 25°C und einem Druck von 0,8 MPA, wie in Beispiel 16, mit Ausnahme dessen, daß man als Mg RX Mg $C_4H_9Cl$ verwendet. Das Molverhältnis Ti $(OC_4H_9)_4$ : Al $(C_2H_5)_3$ : Mg $C_4H_9Cl$ = 1,0:3,7:1,0. Die Ausbeute an Produkt beträgt 767 g/g Ti $(OC_4H_9)_4$ pro Stunde oder 5,4 kg/g Ti pro Stunde. Das Produkt hat folgende Zusammensetzung (in Vol.%): Buten-(1) - 96,2; 3-Methyl-1-buten - 1,1; Hexen-(1) - 0,3; 2-Äthyl-1-buten - 2,4.

Beispiel 23

In einen Reaktor werden 200 ml n-Heptan aufgegeben, das $1,6 \cdot 10^{-3}$ Mol Ti $(OC_4H_9)_4$, $6,4 \cdot 10^{-23}$ Mol Al $(C_2H_5)_3$, $6,4 \cdot 10^{-23}$ Mol Mg $C_4H_9Cl$ enthält und man führt Äthylen zu. Das Molverhältnis Ti $(OC_4H_9)_4$ : Al $(C_2H_5)_3$ : Mg $C_4H_9Cl$ = 1,0:40:4,0.Die Dimerisation erfolgt bei einer Temperatur von 20°C und einem Druck von Äthylen 1,0 MPa. Die Ausbeute an Produkt beträgt 1055 g/g Ti $(OC_4H_9)_4$ pro Stunde oder 7,8 kg/g Ti pro Stunde. Die Zusammensetzung des Produktes (in Vol.%): Buten-(1) - 97,7; 3-Methyl-1-penten - 0,8; Hexen-(1) - 0,5; 2- Äthyl-1-buten - 1,0.

- 10 -

Beispiel 24

Die Dimerisation von Äthylen erfolgt bei einer Temperatur von 60°C und einem Äthylendruck von 3,0 MPa unter Verwendung eines Katalysators, wie in Beispiel 23. Als Lösungsmittel verwendet man Diisopropyläther. Das Molverhältnis Ti $(OC_4H_9)_4$ : Al $(C_2H_5)_3$ : Mg $C_4H_9Cl$ = 1,0:50:1,0. Die Ausbeute an Produkt beträgt 910 g/g Ti $(OC_4H_9)_4$ pro Stunde oder 6,4 kg/g Ti pro Stunde. Die Zusammensetzung des Produktes (in Vol.%): Buten-(1) - 96,7; 3-Methyl-1-penten - 1,4; Hexen-(1) - 0,4; 2-Äthyl-1-buten - 1,5.

Beispiel 25

Die Dimerisation von Äthylen erfolgt bei einer Temperatur von 25°C und einem Druck von Äthylen 1,0 MPa unter Verwendung eines Katalysators wie in Beispiel 23. Das Molverhältnis Ti $(OC_4H_9)_4$ : Al $(C_2H_5)_3$ : Mg $C_4H_9Cl$ = 1,0:100:1,0. Die Ausbeute an Produkt beträgt 1020 g/g Ti $(OC_4H_9)_4$ pro Stunde oder 7,2 kg/g Ti pro Stunde. Die Zusammensetzung des Produktes (in Vol.%): Buten-(1) - 96,9; 3-Methyl-1-penten - 1,5; Hexen-(1) - 0,1; 2- Äthyl-1-buten - 1,5.

Beispiel 26

Die Dimerisation von Äthylen erfolgt bei einer Temperatur von 70°C und einem Äthylendruck von 2 MPA unter Verwendung eines Katalysators, wie in Beispiel 23.

Als Lösungsmittel verwendet man ein Gemisch aus 60% Buten-(1) und 40% Hexene, wie in Beispiel 15. Das Molverhältnis Ti $(OC_4H_9)_4$ : Al $(C_2H_5)_3$ : Mg $C_4H_9Cl$ = 1,0:4,0:1,0. Die Ausbeute an Produkt beträgt 1300 g/g Ti $(OC_4H_9)_4$ pro Stunde oder 9,2 kg/g Ti pro Stunde. Die Zusammensetzung des Produktes (in Vol.%)) Buten-(1) - 97,0; 3-Methyl-1-penten - 1,4; Hexen-(1) - 0,1; 2-Äthyl-1-buten - 1,4; Okten-(1) - 0,1.

Beispiel 27

Die Dimerisation von Äthylen erfolgt bei einer Temperatur von 65°C und einem Äthylendruck von 0,9 MPa unter Verwendung eines Katalysators, wie in Beispiel 23.

Als Lösungsmittel verwendet man Diäthyläther.

Das Molverhältnis Ti $(OC_4H_9)_4$ : Al $(C_2H_5)_3$ : Mg $C_4H_9Cl$ = 1,0:10:2,0. Die Ausbeute an Produkt beträgt 750 g/g Ti $(OC_4H_9)_4$ pro Stunde oder 5,3 kg/g Ti pro Stunde. Die Zusammensetzung des Produktes (in Vol.%): Buten-(1) - 96,0; 3-Methyl-1-penten - 2,0; Hexen-(1) - 0,3; 2-Äthyl-1-buten - 1,6; Okten-(1) - 0,1.

Beispiel 28

Die Dimerisation von Äthylen erfolgt wie in Beispiel 27. Das Molverhältnis $Ti(OC_4H_9)_4 : Al(C_2H_5)_3 : Mg\ C_4H_9Cl = 1,0:25:2,0$. Die Ausbeute an Produkt beträgt 930 g/g $Ti(OC_4H_9)_4$ pro Stunde oder 6,6 kg/g Ti pro Stunde. Die Zusammensetzung des Produktes (in Vol.%): Buten-(1) - 98,7; 3-Methyl-1-penten - 0,7; 2-Äthyl-1-buten - 0,4; Okten-(1) - 0,2.

Beispiel 29

Die Dimerisation erfolgt bei einer Temperatur von 0°C und einem Äthylendruck von 1,5 MPa, wie in Beispiel 23. Die Ausbeute an Produkt beträgt 504 g/g $Ti(OC_4H_9)_4$ pro Stunde oder 3,6 kg/g Ti pro Stunde. Die Zusammensetzung des Produktes (in Vol.%): Buten-(1) - 94,6; 3-Methyl-1-penten - 3,1; 2-Äthyl-1-buten - 1,7; Hexen-(1) - 0,6; Polymer - Spuren.

Gewerbliche Anwendbarkeit

Das erfindungsgemäße Verfahren wird bei der Herstellung von Rohstoffen für die Synthese von Plasten, Divinyl, Isopren beziehungsweise von Zusätzen für Motorenkraftstoffe seine Anwendung finden.

- 12 -

PATENTANSPRUCH

1. Verfahren zur Herstellung von Buten-(1) und/oder Hexenen, das in der Dimerisation von Äthylen und/oder Kodimerisation von Äthylen und Propylen in Gegenwart eines komplexen metallorganischen Katalysators, der Titanalkoholat der Formel Ti $(OR)_4$, worin R - Alkylrest, der von 1 bis 8 Kohlenstoffatomen hat, oder Aryl, Trialkylaluminium der Formel AL $R_3$, worin R für Alkylrest steht, der von 1 bis 6 Kohlenstoffatomen hat, aufweist, bei einer Temperatur von 0 bis 100°C und einem erhöhten Druck im Medium eines organischen Lösungsmittels besteht, d a d u r c h    g e k e n n - z e i c h n e t , daß man einen komplexen metallorganischen Katalysator verwendet, der zusätzlich eine magnesiumorganische Verbindung der Formel $MgR_2$ und/oder MgRX enthält, worin R - Alkylrest ist, der von 1 bis 4 Kohlenstoffatomen hat, X für Br, Cl, J steht.

2. Verfahren nach Anspruch 1, d a d u r c h    g e - k e n n z e i c h n e t , daß man den genannten komplexen metallorganischen Katalysator verwendet, der Ti $(OR)_4$, Al $R_3$, Mg $R_2$ und/oder Mg RX bei einem Molverhältnis 1,0:1,0-100:0,2 - 5,0 enthält.

3. Verfahren nach Anspruch 1 - 2 , d a d u r c h  g e k e n n z e i c h n e t, daß man als kohlenwasserstoffhaltiges organisches Lösungsmittel Buten-(1) und/oder eine Hexenfraktion verwendet.

- 13 -

# VERFAHREN ZUR HERSTELLUNG VON BUTEN-(1) UND/ODER HEXENEN

Z u s a m m e n f a s s u n g

Verfahren zur Herstellung von Buten-(1) und/oder Hexenen besteht in der Dimerisation von Äthylen und/oder Kodimerisation von Äthylen und Propylen in einem organischen Lösungsmittel bei einem erhöhten Druck und bei einer Temperatur von 0 bis 100°C in Gegenwart eines komplexen metallorganischen Katalysators, der Titanalkoholat der Formel $Ti(OR)_4$, worin R - Alkylrest, der von 1 bis 8 Kohlenstoffatomen hat oder Aryl ist, Trialkylaluminium der Formel $Al R_3$, worin R - Alkylrest ist, der von 1 bis 6 Kohlenstoffatomen hat, eine magnesiumorganische Verbindung der Formel $Mg R_2$ und/oder Mg RX, worin R - Alkylrest ist, der von 1 bis 4 Kohlenstoffatomen hat, X für Br, Cl und J steht, aufweist.

# INTERNATIONAL SEARCH REPORT

International Application No PCT/SU 88/00042

## I. CLASSIFICATION OF SUBJECT MATTER (if several classification symbols apply, indicate all) 6

According to International Patent Classification (IPC) or to both National Classification and IPC

IPC⁴    C 07 C 11/08, 11/107, 2/30

## II. FIELDS SEARCHED

Minimum Documentation Searched 7

| Classification System | Classification Symbols |
|---|---|
| IPC⁴ | C 07 C, 2/26-2/32, 11/02-11/107 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | US, A, 4101600, (Viktor Ivanovich Zhukov et al.) 18 July 1978 (18.07.78), see the abstract | 1 |
| A | FR, A1, 2552079, (INSTITUT FRANCAIS DU PETROLE), 22 March 1985, (22.03.85), see the claims<br><br>& JP, A, 60-94923; EP, B1, 0135441; NO, A, 843713 | 1,3 |
| A | SU, A1, 992501, (V.I. Zhukov et al.) 30 January 1983 (30.01.83) see the claims | 1,3 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 15 September 1988 (15.09.88) | 5 November 1988 (05.11.88) |

| International Searching Authority | Signature of Authorized Officer |
|---|---|
| ISA/SU | |

Form PCT/ISA/210 (second sheet) (January 1985)